# EUROPEAN PATENT APPLICATION

(11) **EP 4 043 007 A1**
(43) Date of publication of application: **17.08.2022**
(21) Application number: 20874116.5
(22) Date of filing: 08.10.2020
(51) Int. Cl.: A61K 9/08, A61K 31/197, A61K 45/00, A61K 47/12, A61K 47/18, A61K 47/20, A61K 47/24

(54) **PHARMACEUTICAL COMPOSITION INCLUDING TEMPERATURE-RESPONSIVE IONIC LIQUID**

(30) Priority: 11.10.2019 JP 2019187274
(71) Applicant: Toray Industries, Inc., Tokyo, 103-8666 (JP)
(72) Inventor: MOTOSHIROMIZU, Takahiro, Kamakura-shi, Kanagawa 248-8555 (JP); SUDO, Masafumi, Tokyo 103-8666 (JP); TAKAKI, Suguru, Kamakura-shi, Kanagawa 248-8555 (JP); HIRAKATA, Mikito, Kamakura-shi, Kanagawa 248-8555 (JP); KAJIWARA, Yuri, Kamakura-shi, Kanagawa 248-8555 (JP)
(74) Representative: Kador & Partner PartG mbB
(86) International application number: PCT/JP2020/038097
(87) International publication number: WO 2021/070893

(57) **Abstract**

An object of the present invention is to provide a pharmaceutical composition improved in permeability of a drug to, e.g., the skin and mucous membrane. The present invention provides a pharmaceutical composition containing a drug and a temperature-responsive ionic liquid.

## Description

### Technical Field

The present invention relates to a pharmaceutical composition containing a temperature-responsive ionic liquid.

### Background Art

Oral administration, intravenous administration, transdermal administration and the like are known as an administration route for a pharmaceutical drug and an administration route is appropriately developed in accordance with physical properties, characteristic features and the like of an active ingredient.

In particular, transdermal administration has advantages of improving patient's drug compliance, for example, facilitating elderly persons and patients with dysphagia such as dementia patients and the like to take a drug; avoiding side effects caused by a high bloodconcentration of a pharmaceutical drug administered via the oral route or by injection; and providing stable medicinal effects on busy patients likely having irregular meals since diets rarely affects transdermal administration. Because of these advantages, recently, technical development has proceeded for improving permeability of drugs. However, a water-soluble drug and a drug having a large molecular weight are usually blocked by the stratum corneum of the skin having a high barrier function. Because of this, the skin permeability of such a drug is low and the transdermal absorbability of such a drug is extremely low. Thus, transdermal administration has a problem in that the drug administered is not be sufficiently utilized in a body, different from oral administration and intravenous administration. For the reason, the drugs applicable to transdermal administration are limited. This is a technical problem to be solved.

Up to present, a wide variety of techniques have been developed for improving permeability of a drug to the skin in transdermal administration. In view of drug formulation, adding a transdermal absorption promoting agent is developed. In view of techniques for administration, a technique such as iontophoresis and microneedle is developed. However, a transdermal absorption promoting agent is not commonly used since an optimal transdermal absorption promoting agent varies depending on the drug. In the case where a drug is not compatible with a transdermal absorption promoting agent, a compatibilizing agent is further required. In the techniques for administration, administration gives pain to a patient and principally presents a risk of infection due to a damage given to the skin. These problems still remain unsolved.

Recently, a transdermal absorption technique with an ionic liquid has attracted attention as a technique which can improve compatibility with a drug and solubility of a drug (problems of existing transdermal absorption promoting agents) and by which a transdermal absorption promoting effect may be provided. Since combinations of cations and anions are uncountable, an ionic liquid has a merit of a high degree of freedom in designing its physical properties.

For example, Patent Literature 1 discloses a technique for improving transdermal absorbability of a basic drug such as lidocaine and an acid drug such as etodolac by use of a fatty acid-based ionic liquid.

Patent Literature 2 discloses a technique for improving bioavailability of a drug, more specifically, a technique for improving solubility of taxol by use of an imidazolium ionic liquid, 1-butyl-3-methyl-1H-imidazolium bromide.

Patent Literature 3 discloses a technique for improving skin permeability of a water-soluble drug, more specifically, a transdermal absorption promoting agent containing an equimolar salt of a basic functional group-containing compound (having a molecular weight of 50 to 120 and a melting point of 50 to 350°C) such as choline and guanidine, and an acidic functional group-containing compound (having a LogP of -4 to 7.3).

### Citation List

### Patent Literatures

Patent Literature 1: WO2009/066457
Patent Literature 2: JP 2007-022942A
Patent Literature 3: WO2016/068336

### Summary of Invention

### Technical Problem

The drugs to which the technique of Patent Literature 1 can be applied are limited to acidic drugs or basic drugs. In this respect, the technique is not versatile.

Patent Literature 2 only discloses improving the solubility of taxol (poor soluble medicinal component) by 1-butyl-3-methyl-1H-imidazolium bromide, neither describes nor suggests skin permeability of a drug, and neither discloses nor suggests a temperature-responsive ionic liquid. In addition, since a water-soluble ionic liquid is used, the effect to disturb the lipophilic barrier of the stratum corneum is presumably weak and insufficient.

In Patent Literature 3, the basic functional group-containing compounds supported by specific data disclosed in Examples are limited to choline, histamine, guanidine and imidazole. The literature does not disclose the relationship between a transdermal absorption promoting agent and an ionic liquid, much less disclose or suggest a temperature-responsive ionic liquid.

In the circumstances, an object of the present invention is to provide a pharmaceutical composition improved in drug permeability to e.g., the skin and mucous membrane.

### Solution to Problem

The present inventors conducted intensive studies with a view to attaining the above object. As a result, we found that the drug permeability to, e.g., the skin and mucous membrane, can be improved by use of a temperature-responsive ionic liquid. Based on the finding, the present invention was accomplished.

More specifically, the present invention includes the following [1] to [9].
[1] A pharmaceutical composition comprising a drug and a temperature-responsive ionic liquid.
[2] The pharmaceutical composition according to [1], wherein an anion in the temperature-responsive ionic liquid includes at least one selected from the group consisting of an aromatic carboxylate ion and an aromatic sulfonate ion.
[3] The pharmaceutical composition according to [1] or [2], wherein the anion in the temperature-responsive ionic liquid is an anion represented by the following formula (I) and/or general formula (II): 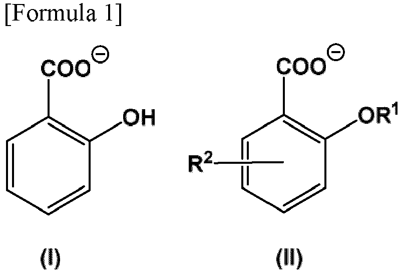 wherein in the formula (II), R¹ represents a hydrogen atom or an acyl group and R² represents an alkyl group optionally substituted with a halogen atom, a halogen atom or a sulfo group.
[4] The pharmaceutical composition according to any one of [1] to [3], wherein the temperature-responsive ionic liquid has a lower critical solution temperature.
[5] The pharmaceutical composition according to [4], wherein the lower critical solution temperature is 40°C or less.
[6] The pharmaceutical composition according to any one of [1] to [5], wherein a cation in the temperature-responsive ionic liquid is a tetraalkylammonium ion or a tetraalkylphosphonium ion.
[7] The pharmaceutical composition according to any one of [1] to [6], which is an external agent.
[8] A temperature-responsive ionic liquid having an anion represented by the following general formula (II): 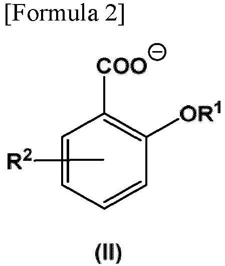 wherein in the formula (II), R¹ represents a hydrogen atom or an acyl group and R² represents an alkyl group optionally substituted with a halogen atom, a halogen atom or a sulfo group.
[9] The temperature-responsive ionic liquid according to [8], having a lower critical solution temperature.

### Advantageous Effects of Invention

A pharmaceutical composition according to the present invention has an excellent drug permeability, and thus, the composition can be applied to various dosage forms.

### Description of Embodiments

The present invention will be more specifically described below.

The pharmaceutical composition of the present invention is characterized by containing a drug serving as an active ingredient and a temperature-responsive ionic liquid.

The temperature-responsive ionic liquid is constituted of a cation and an anion and not particularly limited as long as it has a temperature responsive property. Note that, the melting point of the temperature-responsive ionic liquid is not particularly limited.

The temperature responsive property refers to a property of changing, e.g., a shape and/or nature in response to a temperature (thermal) change; for example, volume changes such as expansion and shrinkage and change in degree of hydrophobicity such as lower critical solution temperature (LCST) and upper critical solution temperature (UCST), are mentioned. The ionic liquid having such a temperature responsive property is referred to as a temperature-responsive ionic liquid.

In the pharmaceutical composition, it is preferable that the temperature-responsive ionic liquid has a lower critical solution temperature (LCST). The lower critical solution temperature refers to the temperature at which, in a temperature-responsive ionic liquid, the degree of hydrophobicity of the temperature-responsive ionic liquid abruptly increases when the temperature of the ionic liquid is raised from a low temperature toward a high temperature. If a temperature-responsive ionic liquid contains water, it does not matter whether or not the temperature-responsive ionic liquid is compatible with water at a temperature lower than the lower critical solution temperature. This is because a temperature-responsive ionic liquid naturally contains a certain amount of water even at a temperature lower than the lower critical solution temperature, thus even if a water phase is separated in the ionic liquid at a temperature lower than the lower critical solution temperature, the degree of hydrophobicity of the temperature-responsive ionic liquid abruptly increases when the liquid is heated up to the lower critical solution temperature or more.

Whether an ionic liquid is a temperature-responsive ionic liquid having a lower critical solution temperature (hereinafter referred to also as LCST-type temperature-responsive ionic liquid) is determined by measuring the absorbance of the liquid at a wavelength within a visible light region while gradually increasing the temperature and observing white turbidity, in other words, an abrupt increase in absorbance at a certain temperature caused by an increase in degree of hydrophobicity. The specific procedure is as follows. First, a mixture of an ionic liquid and water is prepared and the layer state at room temperature is observed. Then, the mixture is placed in a refrigerator of 4°C, sufficiently stirred and allowed to stand still for one hour. The state of the mixture is observed. If the layer state of the ionic liquid-water mixture at room temperature is the same as the layer state at 4°C, the mixture is increased in temperature at a rate of 0.5°C/minute within the range of 20°C to 95°C and the absorbance of the mixture is measured at 450 nm. Based on a change in absorbance, whether the ionic liquid thus measured is LCST-type temperature-responsive ionic liquid or not can be checked. The obtained data of absorbance change can be analyzed by differential calculus according to Tm (nucleic acid melting temperature) analysis program to obtain the lower critical solution temperature. In contrast, if the phase state of the ionic liquid-water mixture at room temperature is different from the state at 4°C, the mixture is increased in temperature at a rate of 0.5°C/minute within the range of 5°C to 40°C and the absorbance of the mixture is measured at 450 nm. Based on a change in absorbance, whether the ionic liquid thus measured is LCST-type temperature-responsive ionic liquid or not can be checked. The reasons why the temperature-responsive ionic liquid having a lower critical solution temperature is preferable are considered as follows; however, the invention of the present application is not limited to the followings. The temperature-responsive ionic liquid having a lower critical solution temperature can efficiently dissolve both a water-soluble drug and a lipophilic drug since the temperature-responsive ionic liquid is amphipathic at a temperature lower than the lower critical solution temperature. In contrast, at a temperature equal to or higher than the lower critical solution temperature, the degree of hydrophobicity of the temperature-responsive ionic liquid increases, and the affinity of the temperature-responsive ionic liquid for the highly lipophilic stratum corneum and mucous membrane increases, with the result that the temperature-responsive ionic liquid efficiently acts on the stratum corneum and mucous membrane. In addition, since the degree of hydrophobicity locally changes, a gradient of a drug concentration occurs within a pharmaceutical composition and between a pharmaceutical composition and the stratum corneum. Consequently, a drug becomes to easily migrate within the base of the pharmaceutical composition and passes through the barrier of the stratum corneum and mucous membrane; in other words, the drug can be improved in permeability.

If an ionic liquid exhibits a temperature responsive property in the environment of around 37°C (as the normal temperature of a human) where a drug is administered, it can be expected that the permeability of the drug is effectively enhanced. In an embodiment, the lower critical solution temperature of a temperature-responsive ionic liquid may be 40°C or less, for example, 35°C or less, 30°C or less, 25°C or less, or 20°C or less. The lower limit value thereof, although it is not particularly limited, is for example, 5°C or more, 10°C or more, or 15°C or more. The upper limit and the lower limit values may be used in any combination. For example, the lower critical solution temperature of a temperature-responsive ionic liquid may be 5°C or more and 40°C or less, 5°C or more and 30°C or less, 5°C or more and 25°C or less, 5°C or more and 20°C or less, 10°C or more and 40°C or less, 10°C or more and 30°C or less, 10°C or more and 25°C or less, 15°C or more and 40°C or less, or 15°C or more and 30°C or less.

Whether an ionic liquid is a temperature-responsive ionic liquid having an upper critical solution temperature (hereinafter referred to also as UCST-type temperature-responsive ionic liquid) or not is determined by measuring the absorbance of the liquid at a wavelength within a visible light region while gradually increasing the temperature, and observing transparency of the liquid, in other words, an abrupt decrease in absorbance at a certain temperature caused by an increase in degree of hydrophilicity. More specifically, whether an ionic liquid is UCST or not can be checked by measuring absorbance in the same manner as in the method for checking the LCST-type temperature-responsive ionic liquid. The upper critical solution temperature can be obtained by analyzing data of absorbance change by differential calculus according to Tm (nucleic acid melting temperature) analysis program.

Any combination of the cation and anion may be used in a temperature-responsive ionic liquid as long as the ionic liquid is temperature responsive. For example, the cation and anion may be used in combination in an equimolar ratio.

Examples of the cation constituting a temperature-responsive ionic liquid, include, but are not limited to, a cation having a nitrogen atom as the ion center, a cation having a phosphorus atom as the ion center, a cation having a sulfur atom as the ion center and a cation having a nitrogen atom and a sulfur atom as the ion center.

Examples of the cation having a nitrogen atom as the ion center include an imidazolium ion, an ammonium ion, a pyridinium ion, a quinolinium ion, a pyrrolidinium ion, a piperidinium ion, piperazinium ion, a morpholinium ion, a pyridazinium ion, a pyrimidinium ion, a pyrazinium ion, a pyrazolium ion, a thiazolium ion, an oxazolium ion, a triazolium ion, a guanidium ion and a 4-aza-1-azonia-bicyclo-[2,2,2]octanium ion. These cations may have a substituent such as an alkyl group at any position. A plurality of substituents may be used.

The cation having a nitrogen atom as the ion center is preferably an imidazolium ion, an ammonium ion, a pyridinium ion, a pyrrolidinium ion or a piperidinium ion.

Specific examples of the imidazolium ion include a 1-methylimidazolium ion, a 1-ethylimidazolium ion, a 1-propylimidazolium ion, a 1-butylimidazolium ion, a 1-butyl-3-methylimidazolium ion, a 1-ethyl-3-methylimidazolium ion, a 1-allyl-3-methylimidazolium ion, a 1,3-diallylimidazolium ion, a 1-benzyl-3-methylimidazolium ion, a 1-methyl-3-octylimidazolium ion, a 1-ethyl-2,3-dimethylimidazolium ion, a 1-butyl-2,3-dimethylimidazolium ion, a 1,2-dimethyl-3-propylimidazolium ion, a 1-cyanopropyl-3-methylimidazolium ion, a 1,3-biscyanomethylimidazolium ion, a 1,3-bis(3-cyanopropyl)imidazolium ion, a 1-(2-hydroxyethyl)-3-methylimidazolium ion, a 1-methoxyethyl-3-methylimidazolium ion, a 1-[2-(2-methoxyethoxy)-ethyl]-3-methylimidazolium ion, a 1,3-diethoxyimidazolium ion, a 1,3-dimethoxyimidazolium ion, a 1,3-dihydroxyimidazolium ion, a 1-methyl-3-(3,3,4,4,5,5,6,6,7,7,8,8,8-tridecafluorooctylimidazolium ion and a 1-methyl-3-[(triethoxysilyl)propyl] imidazolium ion.

Specific examples of the ammonium ion include a tetraalkylammonium ion such as a tetramethylammonium ion, a tetraethylammonium ion, a tetrapropylammonium ion, a tetrabutylammonium ion, a tetrahexylammonium ion and a trihexyltetradecylammonium ion; a (2-hydroxyethyl)trimethylammonium ion, a N,N-diethyl-N-(2-methoxyethyl)-N-methylammonium ion, a tris(2-hydroxyethyl)methylammonium ion, atrimethyl(1H,1H,2H,2H-heptadecafluorodecyl)ammonium ion, a trimethyl-(4-vinylbenzyl)ammonium ion, a tributyl-(4-vinylbenzyl)ammonium ion, a 2-(methacryloyloxy)ethyltrimethylammonium ion, a benzyldimethyl(octyl)ammonium ion and a N,N-dimethyl-N-(2-phenoxyethyl)-1-dodecylammonium ion.

Specific examples of the pyridinium ion include a 1-ethylpyridinium ion, 1-butylpyridinium ion, a 1-(3-hydroxypropyl)pyridinium ion, a 1-ethyl-3-methylpyridinium ion, a 1-butyl-3-methylpyridinium ion, a 1-butyl-4-methylpyridinium ion and a 1-(3-cyanopropyl)pyridinium ion.

Specific examples of the pyrrolidinium ion include a 1-methyl-1-propylpyrrolidinium ion, a 1-butyl-1-methylpyrrolidinium ion, a 1-(2-hydroxyethyl)-1-methylpyrrolidinium ion and a 1-ethyl-1-methylpyrrolidinium ion.

Specific examples of the piperidinium ion include a 1-methyl-1-propylpiperidinium ion, a 1-butyl-1-methylpiperidinium ion, a 1-(2-hydroxyethyl)-1-methylpiperidinium ion and a 1-ethyl-1-methylpiperidinium ion.

A cation having a phosphorus atom as the ion center is generally called as a phosphonium ion. Examples of the phosphonium ion include a tetraalkylphosphonium ion such as a tetrapropylphosphonium ion, a tetrabutylphosphonium ion, a tetrahexylphosphonium ion, a trihexyltetradecylphosphonium ion, a triphenylmethylphosphonium ion, a triisobutylmethylphosphonium ion, a triethylmethylphosphonium ion, a tributylmethylphosphonium ion and a tributylhexadecylphosphonium ion; a (2-cyanoethyl)triethylphosphonium ion, a (3-chloropropyl)trioctylphosphonium ion, a tributyl(4-vinylbenzyl)phosphonium ion and a 3-(triphenylphosphonio)propane-1-sulfonate ion.

A cation having a sulfur atom as the ion center is generally called as a sulfonium ion. Examples of the sulfonium ion include a triethylsulfonium ion, a tributylsulfonium ion, a 1-ethyltetrahydrothiophenium ion and a 1-butyltetrahydrothiophenium ion.

As an embodiment of the cation constituting a temperature-responsive ionic liquid having an appropriate lipid solubility, simultaneously with a temperature responsive property and a drug solubility, an ammonium ion or a phosphonium ion is preferable, and a tetraalkylammonium ion or a tetraalkylphosphonium ion is more preferable; and in one embodiment, a tetraalkylammonium ion (a tetrabutylammonium ion, a tetrapentylammonium ion, a tetrahexylammonium ion, a tetraheptylammonium ion, a tetraoctylammonium ion and a trioctylmethylammonium ion), or a tetraalkylphosphonium ion (a tetrabutylphosphonium ion, a tetrapentylphosphonium ion, a tetraoctylphosphonium ion, a tributylhexylphosphonium ion, a tributylheptylphosphonium ion, a tributyloctylphosphonium ion, a tributyldodecylphosphonium ion, a tributyl tridecylphosphonium ion, a tributylpentadecylphosphonium ion, a tributylhexadecylphosphonium ion and a trihexyltetradecylphosphonium ion), having 13 or more and 35 or less carbon atoms as a total number of carbon atoms of four alkyl groups may be mentioned. In each of the tetraalkylammonium ion and tetraalkylphosphonium ion, the four alkyl groups may be the same or different. In each of the tetraalkylammonium ion and tetraalkylphosphonium ion, the value of CLogP showing the degree of hydrophobicity of a cation is preferably larger than -1.0.

Examples of the anion constituting a temperature-responsive ionic liquid include, but are not limited to, a halide ion, an imide ion such as a bis(trifluoromethylsulfonyl)imide (ion), a carboxylate ion, a sulfonate ion, a phosphate ion, a phosphonate ion, a phosphinate ion and an amino acid ion. These anions may have a substituent represented by an alkyl group and an aryl group at any position. A plurality of substituents may be used.

As the anion constituting a temperature-responsive ionic liquid, it is preferable to include at least one selected from the group consisting of an aromatic carboxylate ion and an aromatic sulfonate ion.

Examples of the aromatic carboxylate ion include substituted and unsubstituted benzoate ions, for example, a benzoate ion and a benzoate ion substituted with an optionally substituted alkyl group, such as a toluic acid ion or a trifluoromethylbenzoate ion; a halogenated benzoate ion such as a fluorobenzoate ion, a chlorobenzoate ion, a bromobenzoate ion or an iodine benzoate ion; an acylated benzoate ion such as a formylbenzoate ion or an acetylbenzoate ion; a hydroxybenzoate ion; an aminobenzoate ion; an alkoxylated benzoate ion such as a methoxybenzoate ion; a sulfobenzoate ion; and a substituted or unsubstituted polycyclic aromatic carboxylate ion such as a naphthalene carboxylate ion or an anthracene carboxylate ion. The position of a substituent of an aromatic carboxylate ion is not limited, and the number and types of substituents are not particularly limited, as long as the ion constituting a temperature-responsive ionic liquid. If a substituent such as a hydroxy group and an amino group is contained, the hydroxy group and amino group may be substituted further with an acyl group or the like.

Examples of the aromatic sulfonate ion include a substituted and unsubstituted benzene sulfonate ions, for example, a benzenesulfonate ion, and a benzenesulfonate ion with an optionally substituted alkyl group, such as a toluenesulfonate ion, a xylenesulfonate ion or a trichloromethylsulfonate ion; a halogenated benzenesulfonate ion such as a fluorobenzenesulfonate ion, a chlorobenzenesulfonate ion, a bromobenzenesulfonate ion or an iodobenzenesulfonate ion; a hydroxybenzene sulfonate ion; an aminobenzene sulfonate ion; and an alkoxylated benzenesulfonate ion such as a methoxybenzene sulfonate ion. The position of a substituent of an aromatic sulfonate ion is not limited, and the number and types of substituents are not particularly limited as long as the ion constituting a temperature-responsive ionic liquid. If a substituent such as a hydroxy group and an amino group is contained, the hydroxy group and amino group may be substituted further with an acyl group or the like.

Examples of a preferable anion constituting a temperature-responsive ionic liquid include an anion derived from salicylic acid or a salicylic acid derivative. Examples of the salicylic acid derivative include a compound having a substituent on a benzene ring of salicylic acid and a compound having a substituent at a phenolic hydroxy group. In an embodiment, examples of the anion constituting the temperature-responsive ionic liquid is an anion represented, for example, by the following formula (I) and/or general formula (II):

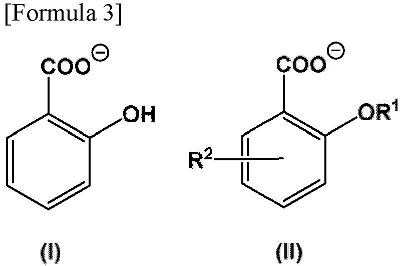

In the formula (II), R¹ represents a hydrogen atom or an acyl group. Examples of the acyl group used herein include an acetyl group.

In the formula (II), R² represents an alkyl group optionally substituted with a halogen atom, a halogen atom or a sulfo group. Examples of the alkyl group optionally substituted with a halogen atom used herein include, but are not limited to, a methyl group, an ethyl group, a propyl group, a butyl group, a fluoromethyl group, a difluoromethyl group, a trifluoromethyl group and a 2-fluoroethyl group. Examples of the halogen atom include a fluorine atom, a chlorine atom, a bromine atom and an iodine atom. The position of R² is not limited.

The temperature-responsive ionic liquid to be used in the pharmaceutical composition of the present invention include a temperature-responsive ionic liquid containing an ammonium ion (for example, tetraalkylammonium ion) and an aromatic carboxylate ion (for example, an anion derived from salicylic acid or a salicylic acid derivative); a temperature-responsive ionic liquid containing an ammonium ion (for example, tetraalkylammonium ion) and an aromatic sulfonate ion; a temperature-responsive ionic liquid containing a phosphonium ion (for example, tetraalkylphosphonium ion) and an aromatic carboxylate ion (for example, an anion derived from salicylic acid or a salicylic acid derivative); and a temperature-responsive ionic liquid containing a phosphonium ion (for example, tetraalkylphosphonium ion) and an aromatic sulfonate ion. Specific examples of the temperature-responsive ionic liquid include a tetrabutylammonium salicylate, tetrabutylammonium 4-(trifluoromethyl)salicylate, tetrabutylphosphonium salicylate, tetrabutylphosphonium 4-(trifluoromethyl)salicylate, tetrabutylphosphonium acetylsalicylate, tetrabutylphosphonium 5-bromosalicylate, tetrabutylphosphonium 4-chlorosalicylate, tetrabutylphosphonium 5-chlorosalicylate, tetrabutylphosphonium 5-iodosalicylate, tetrabutylphosphonium 3-methylsalicylate, tetrabutylphosphonium 4-methylsalicylate, tetrabutylphosphonium 3-carboxy-4-hydroxybenzenesulfonate, tetrabutylphosphonium p-toluenesulfonate, tetrabutylphosphonium m-xylene-4-sulfonate, tetrabutylphosphonium 4-chlorobenzenesulfonate, tetrabutylphosphonium 4-hydroxybenzenesulfonate, and tetrabutylphosphonium 4-aminotoluene-3-sulfonate.

Examples of the temperature-responsive ionic liquid to be used in the pharmaceutical composition of the present invention include not only an ionic liquid exhibiting a temperature responsive property when used alone, but also a combination of ionic liquids exhibiting a temperature responsive property when used in combination as well as a temperature-responsive ionic liquid containing at least two types of cations and/or anions. Herein, an ionic liquid that does not exhibit a temperature responsive property when used alone may be used in combination with a temperature-responsive ionic liquid as long as the resultant ionic liquid exhibits a temperature responsive property.

For example, an ionic liquid, tetrabutylphosphonium 4-aminosalicylate, does not exhibit a temperature responsive property when used alone; however, if the ionic liquid is mixed with tetrabutylphosphonium salicylate in an equimolar ratio, the resultant ionic liquid mixture can exhibit a temperature responsive property. The value of the lower critical solution temperature can be arbitrarily controlled by appropriately changing the mixing ratio of the two ionic liquids.

The value of the lower critical solution temperature can be arbitrarily controlled further by a method of mixing two or more types of temperature-responsive ionic liquids in an arbitrary ratio or by a method of using a single type of cation and two or more types of anions; two or more types of cations and a single type of anion; or two or more types of cations and two or more types of anions for synthesizing a temperature-responsive ionic liquid. For example, tetrabutylphosphonium hydroxide (one mole) is mixed with salicylic acid and 4-(trifluoromethyl)salicylic acid (one mole in total) to obtain a temperature-responsive ionic liquid containing two types of anions.

A temperature-responsive ionic liquid may be a commercially available product or can be synthesized in accordance with a synthesis method generally used for preparing an ionic liquid. A temperature-responsive ionic liquid being an ammonium salt is produced, for example, by a method (anion exchange method), in which an ammonium salt obtained by reacting an amine with, e.g., a halogenated alkyl compound, is subjected to anion exchange using a metal salt followed by a purification process, or a method (neutralization method) in which an amine and an acid are directly reacted and neutralized; however, the production method of the temperature-responsive ionic liquid is not limited to these. A temperature-responsive ionic liquid being a phosphonium salt can be synthesized by not only the anion exchange method but also the neutralization method. In the case of the neutralization method, phosphonium hydroxide is directly reacted with an acid and then neutralized to obtain a desired phosphonium salt.

The drug to be used in the pharmaceutical composition of the present invention is not particularly limited and appropriately selected from drugs (active ingredients) commonly known.

Examples of the drug include, but are not limited to, a steroidal anti-inflammatory agent such as prednisolone, dexamethasone, hydrocortisone, fluocinolone acetonide, betamethasone valerate, betamethasone dipropionate, clobetasone butyrate and prednisolone succinate; a nonsteroidal anti-inflammatory drug such as indomethacin, diclofenac, ibuprofen, ketoprofen, flufenamic acid, ketorolac, flurbiprofen, felbinac, suprofen, pranoprofen, tiaprofen and loxoprofen, and ester derivatives thereof; an antiallergic agent such as tranilast, azelastine, ketotifen, ibudilast, oxatomide and emedastine; an antihistamine agent such as diphenhydramine, chlorpheniramine, promethazine and tripelennamine; a central nervous system agent such as chlorpromazine, nitrazepam, diazepam, phenobarbital and reserpine; a hormonal agent such as insulin, testosterone, norethisterone, methyltestosterone, progesterone and estradiol; an antihypertension agent such as clonidine, reserpine and guanethidine sulfate; a cardiac stimulant such as digitoxin and digoxin; an antiarrhythmic agent such as propranolol hydrochloride, procainamide hydrochloride, ajmaline, pindolol and tulobuterol hydrochloride; a coronary vasodilator such as nitroglycerin, isosorbide dinitrate, papaverine hydrochloride and nifedipine; a local anesthetic such as lidocaine, benzocaine, procaine hydrochloride and tetracaine; a painkiller such as morphine, aspirin, codeine, tramadol, pregabalin, mirogabalin, acetanilide, aminopyrine and etodolac; a skeletal muscle relaxant such as eperisone, tizanidine, tolperisone, inaperisone and pridinol mesylate; an antifungal agent such as acetphenylamine, nitrofurazone, pentamycin, naphthiomate, miconazole, omoconazole, clotrimazole, butenafine hydrochloride and bifonazole; an anti-malignant tumor agent such as 5-fluorouracil, busulfan, actinomycin, bleomycin and mitomycin; an agent for dysuria such as terodiline hydrochloride and oxybutynin hydrochloride; an antiepileptic drug such as nitrazepam and meprobamate; a Parkinson's disease therapeutic drug such as rotigotine, ropinirole, chlorzoxazone and levodopa; an antipsychotic such as blonanserin; a dementia therapeutic drug such as donepezil and rivastigmine; a stop smoking aids such as nicotine; a vitamin; and a prostaglandin. These drugs may be pharmacologically acceptable salts and solvates thereof. Examples of the pharmacologically acceptable salts include inorganic acid salts and organic acid salts. Examples of the inorganic acid salts include a hydrochloride, a sulfate, a nitrate, a hydrobromide, a hydroiodide and a phosphate. Examples of the organic acid salts include an oxalate, a malonate, a citrate, a fumarate, a lactate, a malate, a succinate, a tartrate, an acetate, a trifluoroacetate, a maleate, a gluconate, a benzoate, an ascorbate, a glutarate, a mandelate, a phthalate, a methanesulfonate, an ethanesulfonate, a benzenesulfonate, a p-toluenesulfonate, a camphorsulfonate, an aspartate, a glutamate and a cinnamate. As the solvate, for example, a hydrate is mentioned.

The molecular weights of the drugs, although they are not particularly limited, are for example, 100 to 1000, 150 to 600, 150 to 500, 150 to 400 or 150 to 350.

Since the pharmaceutical composition of the present invention contains a temperature-responsive ionic liquid, not only the solubility of a drug but also the permeability of the drug to the epidermis as well as the mucous membrane can be improved. Because of these properties, commonly known oral administration and parenteral administration (routes) such as nasal administration, intravenous administration, transpulmonary administration and transdermal administration, can be used.

Examples of dosage form of the pharmaceutical composition of the present invention for oral administration include tablets (sugar-coated tablets and film coated tablets), pills, granules, powders, capsules (including soft capsules and microcapsules), syrups, emulsions and suspensions.

A preparation of oral dosage forms can be produced in accordance with a method commonly known in the pharmaceutical field, more specifically, by appropriately adding pharmacologically acceptable additives commonly used in the pharmaceutical field, such as an excipient, a binder, a lubricant, a disintegrant, a sweetener, a surfactant, a suspension agent and/or an emulsifier.

Examples of dosage form of the pharmaceutical composition of the present invention for parenteral administration include an intranasal drug, an eye drop, an injection, an impregnating agent, an intravenous drip, an external agent and a suppository. Since the pharmaceutical composition of the present invention has a permeation promoting effect on the stratum corneum, the dosage form thereof is preferably a non-invasive administration form, an external agent.

The dosage form of the external agent that can be used may be any one of the dosage forms commonly used, such as an ointment, a cream, a gel, a gel cream, a lotion, a spray, a poultice, a tape agent and a reservoir patch.

The external agent can be prepared in accordance with a method commonly known in the pharmaceutical field, more specifically by appropriately adding pharmacologically acceptable additives commonly used in the pharmaceutical field. A typical dosage form such as a poultice and a tape agent will be described below; however, the dosage form is not limited to these.

In preparation of a poultice, a pharmacologically acceptable additive such as a water-soluble polymer and a polyhydric alcohol can be used.

Examples of the water-soluble polymer include gelatin, casein, pullulan, dextran, sodium alginate, soluble starch, carboxy starch, dextrin, carboxymethyl cellulose, sodium carboxymethyl cellulose, methyl cellulose, ethyl cellulose, hydroxyethyl cellulose, polyvinyl alcohol, polyethylene oxide, polyacrylic acid, polyacrylamide, sodium polyacrylate, polyvinylpyrrolidone, carboxy vinyl polymer, polyvinyl ether, methoxyethylene-maleic anhydride copolymer, isobutylene-maleic anhydride copolymer, N-vinyl acetamide, and copolymer of N-vinyl acetamide and acrylic acid and/or a acrylate.

Examples of the polyhydric alcohol include polyethylene glycol, propylene glycol, dipropylene glycol, polypropylene glycol, 1.3-butylene glycol, 1.4-butylene glycol, isobutylene glycol, glycerin, diglycerin and sorbitol.

Examples of the pharmacologically acceptable additive that can be used for preparing a tape agent include an adhesive base and a tackifier.

The adhesive base can be appropriately selected from commonly known adhesive bases in consideration of safety to the skin, medicinal component releasing characteristic and adhesiveness to the skin. Examples of the adhesive base include an acrylic adhesive, a rubber adhesive and a silicone adhesive.

Examples of the acrylic adhesive include alkyl (meth)acrylate homopolymer, copolymer thereof, and copolymer of alkyl (meth)acrylate and functionalgroup-containing monomer.

Examples of the rubber adhesive include natural rubber, synthetic isoprene rubber, polyisobutylene, polyvinyl ether, polyurethane, polyisoprene, polybutadiene, styrene-butadiene copolymer, styrene-isoprene copolymer and styrene-isoprene-styrene block copolymer

Examples of the silicone adhesive include polyorganosiloxane and polydimethylsiloxane.

Examples of the tackifier include a rosin-base adhesive such as rosin, hydrogenated, disproportionated, polymerized and esterified rosin derivatives, a terpene resin such as α-pinene and β-pinene, a terpene-phenol resin, an aliphatic petroleum resin, an aromatic petroleum resin, an alicyclic petroleum resin, copolymerized petroleum resin, an alkyl-phenyl resin and a xylene resin.

The amount of the temperature-responsive ionic liquid contained in the pharmaceutical composition of the present invention, although it is not particularly limited, is preferably an amount sufficient to improve the solubility of a drug and fall within the range of, for example, 1 to 1000000 wt% relative to the weight of the drug. If the amount of the temperature-responsive ionic liquid exceeds 10 wt% relative to the weight of a pharmaceutical composition, the temperature-responsive ionic liquid is encapsulated in, e.g., soft capsule or powdered by use of appropriate porous body to prepare the pharmaceutical composition.

The present invention provides a temperature-responsive ionic liquid that can be used in the pharmaceutical composition of the present invention.

In an embodiment, a temperature-responsive ionic liquid having an anion represented by the following general formula (II) is mentioned.

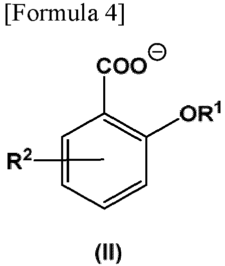

In the formula (II), R¹ represents a hydrogen atom or an acyl group and R² represents an alkyl group optionally substituted with a halogen atom, a halogen atom or a sulfo group.

The number of the types of anions represented by the general formula (II) may be single or two or more.

In an embodiment, examples of the temperature-responsive ionic liquid include, but are not limited to, a temperature-responsive ionic liquid containing an anion represented by the general formula (II), an ammonium ion (for example, tetrabutylammonium ion) or a phosphonium ion (for example, tetraalkylphosphonium ion). Specific examples of the temperature-responsive ionic liquid include a tetrabutylammonium 4-(trifluoromethyl)salicylate, tetrabutylphosphonium 4-(trifluoromethyl)salicylate, tetrabutylphosphonium acetylsalicylate, tetrabutylphosphonium 5-bromosalicylate, tetrabutylphosphonium 4-chlorosalicylate, tetrabutylphosphonium 5-chlorosalicylate, tetrabutylphosphonium 5-iodosalicylate, tetrabutylphosphonium 3-methylsalicylate, tetrabutylphosphonium 4-methylsalicylate, and tetrabutylphosphonium 3-carboxy-4-hydroxybenzenesulfonate.

Two or more of anions may be used in the temperature-responsive ionic liquid having the anion represented by the general formula (II). For example, two types of components serving as the anion represented by the general formula (II) may be used for synthesis. Alternatively, two or more types of temperature-responsive ionic liquids having an anion represented by the general formula (II) may be mixed. Specific examples thereof include, but are not limited to, tetrabutylammonium salicylate/4-(trifluoromethyl)salicylate, tetrabutylphosphonium salicylate/4-(trifluoromethyl)salicylate. In addition, examples thereof include a temperature-responsive ionic liquid, which is obtained by mixing an ionic liquid having a temperature-responsive property and an ionic liquid having no temperature responsive property (such as an ionic liquid obtained by mixing tetrabutylphosphonium 3-methylsalicylate and tetrabutylphosphonium 4-aminosalicylate, an ionic liquid obtained by mixing tetrabutylphosphonium 4-methylsalicylate and tetrabutylphosphonium 4-aminosalicylate).

It is preferable that the temperature-responsive ionic liquid has a lower critical solution temperature. In an embodiment, the lower critical solution temperature of a temperature-responsive ionic liquid may be 40°C or less, for example, 35°C or less, 25°C or less, or 20°C or less. The lower limit thereof, although it is not particularly limited, is for example, 5°C or more, 10°C or more, or 15°C or more. The combination of the upper limit and the lower limit thereof can be arbitrarily set. For example, the lower critical solution temperature of a temperature-responsive ionic liquid is 5°C or more and 40°C or less, 5°C or more and 30°C or less, 5°C or more and 25°C or less, 5°C or more and 20°C or less, 10°C or more and 40°C or less, 10°C or more and 30°C or less, 10°C or more and 25°C or less, 15°C or more and 40°C or less, or 15°C or more and 30°C or less.

Not only the solubility of a drug but also the permeability of a drug to the epidermis as well as the mucous membrane can be improved by the temperature-responsive ionic liquid. Because of the properties, the temperature-responsive ionic liquid can be used as a solubilizer, a dissolution aid and a transdermal absorption promoting agent.

### Examples

Now, the present invention will be more specifically described by way of Reference Examples and Examples; however, the present invention is not limited to these.

For compounds used for synthesis of the compounds of Synthesis Examples and Reference Examples for which no synthesis methods are described, commercially available compounds were used. Symbol "%" used for a yield represents mol/mol%; "%" used for a solvent involved in column chromatography or high performance liquid chromatography represents vol%; and "%" used for other cases represents wt%, unless otherwise specified. The names of the solvents shown in NMR data represent the solvents used in the measurement. The NMR spectrum measured at 400 MHz was obtained by use of JNM-ECS400 type nuclear magnetic resonance device manufactured by JEOL Ltd. A chemical shift was expressed by δ (unit: ppm) based on tetramethylsilane and signals were shown by s (single line), d (double line), t (triple line), q (quadruple line), m (multiple lines) or br (broad), or in combination of these. If a proton of, e.g., a hydroxy group or an amino group, shows a very moderate peak, the peak is not described. IR spectrum was measured by ATR method using FT/IR-6800 manufactured by JASCO Corporation. Molecular weights were measured by LC-MS/MS (LC: Nexera 20A/30A manufactured by Shimadzu Corporation, MS/MS: QTRAP-5500 manufactured by SCIEX for detection of cations; LC: ACUQUITY UPLC I-Class manufactured by Waters, MS/MS: API-5000 manufactured by SCIEX for detection of anions). The amounts of various drugs permeated were measured by use of LC-MS/MS (LC: Nexera 20A/30A manufactured by Shimadzu Corporation, MS/MS: QTRAP-5500 manufactured by SCIEX). As reaction reagents, commercially available reagents were used without purification, unless otherwise specified.

### (Synthesis Example 1) Synthesis of tetrabutylphosphonium 4-(trifluoromethyl)salicylate:

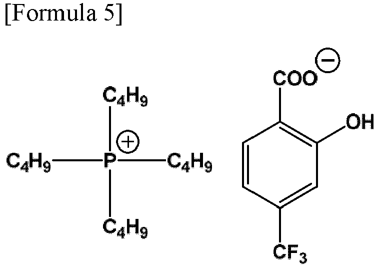

A 40% aqueous solution of tetrabutylphosphonium hydroxide (6.91 g (10 mmol)) and 4-(trifluoromethyl)salicylic acid (2.06 g (10 mmol)) were individually weighed and placed in a glass vial. The reaction solution was stirred at room temperature for two hours. After confirming that 4-(trifluoromethyl)salicylic acid was completely dissolved, the reaction was terminated. As a result, a mixture of tetrabutylphosphonium 4-(trifluoromethyl)salicylate and water (hereinafter referred to as Synthesis Example 1-sample) was quantitatively obtained. Synthesis Example 1-sample was used in Example 1, Example 2, Example 4 and Example 5 described later.

A part of Synthesis Example 1-sample obtained was taken and dichloromethane was added thereto to extract an ionic liquid. Thereafter, the organic layer was dried over anhydrous magnesium sulfate and then concentrated to obtain tetrabutylphosphonium 4-(trifluoromethyl)salicylate (hereinafter referred to as Synthesis Example 1-compound) as a light yellow liquid. Synthesis Example 1-compound obtained was subjected to ¹H-NMR, FT-IR and MS measurements. The measurement results are as follows. It was confirmed that a desired ionic liquid was obtained.

¹H-NMR (CDCl₃) δ: 8.01 (1H, d, J = 8.0 Hz), 7.06 (1H, s), 6.94 (1H, d, J = 8.0 Hz), 2.26-2.18 (8H, m), 1.51-1.44 (16H, m), 0.94 (12H, t, J = 8.0 Hz).

FT-IR: 3390 cm⁻¹ (OH stretching vibration), 1592 cm⁻¹ (C=O stretching vibration)

MS: [M]⁺ = 259, [M-H]⁻ = 205

### (Synthesis Example 2) Synthesis of tetrabutylphosphonium 5-bromosalicylate:

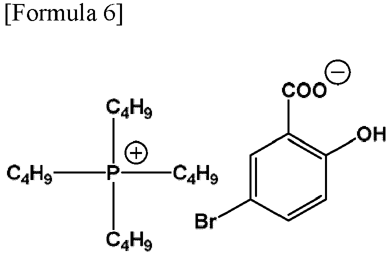

A 40% aqueous solution of tetrabutylphosphonium hydroxide (6.91 g (10 mmol)) and 5-bromosalicylic acid (2.17 g (10 mmol)) were individually weighed and placed in a glass vial. The reaction solution was stirred at room temperature for two hours. After confirming that 5-bromosalicylic acid was completely dissolved, the reaction was terminated. As a result, a mixture of tetrabutylphosphonium 5-bromo salicylate and water (hereinafter referred to as Synthesis Example 2-sample) was quantitatively obtained. Synthesis Example 2-sample was used in Example 1 described later.

A part of Synthesis Example 2-sample obtained was taken and dichloromethane was added thereto to extract an ionic liquid. Thereafter, the organic layer was dried over anhydrous magnesium sulfate and then concentrated to obtain tetrabutylphosphonium 5-bromosalicylate (hereinafter referred to as Synthesis Example 2-compound) as a light yellow liquid. Synthesis Example 2-compound obtained was subj ected to ¹H-NMR, FT-IR and MS measurements. The measurement results are as follows. It was confirmed that a desired ionic liquid was obtained.

¹H-NMR (CDCl₃) δ: 8.01 (1H, dd, J = 2.9, 1.3 Hz), 7.28 (1H, dd, J = 8.3, 2.9 Hz), 6.72 (1H, dd, J = 8.3, 1.3 Hz), 2.23-2.16 (8H, m), 1.51-1.43 (16H, m), 0.94 (12H, t, J = 7.8 Hz).

FT-IR: 3398 cm⁻¹ (OH stretching vibration), 1577 cm⁻¹ (C=O stretching vibration)

MS: [M]⁺ = 259, [M-H]⁻ = 217

### (Synthesis Example 3) Synthesis of tetrabutylphosphonium 3-methylsalicylate:

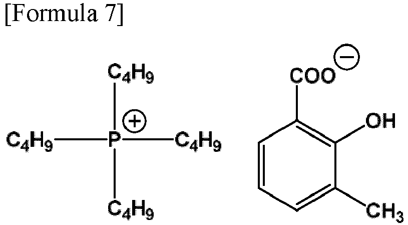

A 40% aqueous solution of tetrabutylphosphonium hydroxide (6.91 g (10 mmol)) and 3-methylsalicylic acid (1.52 g (10 mmol)) were individually weighed and placed in a glass vial. The reaction solution was stirred at room temperature for two hours. After confirming that 3-methylsalicylic acid was completely dissolved, the reaction was terminated. As a result, a mixture of tetrabutylphosphonium 3-methylsalicylate and water (hereinafter referred to as Synthesis Example 3-sample) was quantitatively obtained. Synthesis Example 3-sample was used in Example 1 described later.

A part of Synthesis Example 3-sample obtained was taken and dichloromethane was added thereto to extract an ionic liquid. Thereafter, the organic layer was dried over anhydrous magnesium sulfate and then concentrated to obtain tetrabutylphosphonium 3-methylsalicylate (hereinafter referred to as Synthesis Example 3-compound) as a light yellow liquid. Synthesis Example 3-compound obtained was subjected to ¹H-NMR, FT-IR and MS measurements. The measurement results are as follows. It was confirmed that a desired ionic liquid was obtained.

¹H-NMR (CDCl₃) δ: 7.77 (1H, d, J = 7.7 Hz), 7.09 (1H, d, J = 7.7 Hz), 6.61 (1H, t, J = 7.7 Hz), 2.23 (3H, s) 2.22-2.13 (8H, m), 1.53-1.36 (16H, m), 0.93 (12H, t, J = 8.0 Hz).

FT-IR: 3398 cm⁻¹ (OH stretching vibration), 1577 cm⁻¹ (C=O stretching vibration)

MS: [M]⁺ = 259, [M-H]⁻ = 151

### (Synthesis Example 4) Synthesis of tetrabutylphosphonium 4-methylsalicylate:

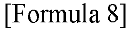

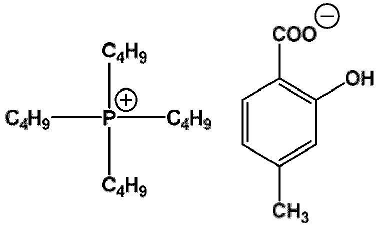

A 40% aqueous solution of tetrabutylphosphonium hydroxide (6.91 g (10 mmol)) and 4-methylsalicylic acid (1.52 g (10 mmol)) were individually weighed and placed in a glass vial. The reaction solution was stirred at room temperature for two hours. After confirming that 4-methylsalicylic acid was completely dissolved, the reaction was terminated. As a result, a mixture of tetrabutylphosphonium 4-methylsalicylate and water (hereinafter referred to as Synthesis Example 4-sample) was quantitatively obtained. Synthesis Example 4-sample was used in Example 1 described later.

A part of Synthesis Example 4-sample obtained was taken and dichloromethane was added thereto to extract an ionic liquid. Thereafter, the organic layer was dried over anhydrous magnesium sulfate and then concentrated to obtain tetrabutylphosphonium 4-methylsalicylate (hereinafter referred to as Synthesis Example 4-compound) as a light yellow liquid. Synthesis Example 4-compound obtained was subj ected to ¹H-NMR, FT-IR and MS measurements. The measurement results are as follows. It was confirmed that a desired ionic liquid was obtained.

¹H-NMR (CDCl₃) δ: 7.78 (1H, d, J = 7.8 Hz), 6.63 (1H, d, J = 1.5 Hz), 6.53 (1H, dd, J = 7.8, 1.5 Hz), 2.45 (1H, br), 2.27 (3H, s), 2.23-2.14 (8H, m), 1.51-1.39 (16H, m), 0.94 (12H, t, J = 7.4 Hz).

FT-IR: 3398 cm⁻¹ (OH stretching vibration), 1581 cm⁻¹ (C=O stretching vibration)

MS: [M]⁺ = 259, [M-H]⁻ = 151

### (Synthesis Example 5) Synthesis of tetrabutylphosphonium 4-chlorosalicylate:

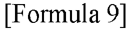

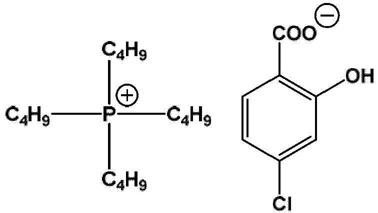

A 40% aqueous solution of tetrabutylphosphonium hydroxide (6.91 g (10 mmol)) and 4-chlorosalicylic acid (1.73 g (10 mmol)) were individually weighed and placed in a glass vial. The reaction solution was stirred at room temperature for two hours. After confirming that 4-chlorosalicylic acid was completely dissolved, the reaction was terminated. As a result, a mixture of tetrabutylphosphonium 4-chlorosalicylate and water (hereinafter referred to as Synthesis Example 5-sample) was quantitatively obtained. Synthesis Example 5-sample was used in Example 1 described later.

A part of Synthesis Example 5-sample obtained was taken and dichloromethane was added thereto to extract an ionic liquid. Thereafter, the organic layer was dried over anhydrous magnesium sulfate and then concentrated to obtain tetrabutylphosphonium 4-chlorosalicylate (hereinafter referred to as Synthesis Example 5-compound) as an orange liquid. Synthesis Example 5-compound obtained was subjected to ¹H-NMR, FT-IR and MS measurements. The measurement results are as follows. It was confirmed that a desired ionic liquid was obtained.

¹H-NMR (CDCl₃) δ: 7.83 (1H, d, J = 8.0 Hz), 6.82 (1H, d, J = 2.5 Hz), 6.53 (1H, dd, J = 8.0, 2.5 Hz), 2.24-2.16 (8H, m), 1.51-1.41 (16H, m), 0.95 (12H, t, J = 7.2 Hz).

FT-IR: 3394 cm⁻¹ (OH stretching vibration), 1574 cm⁻¹ (C=O stretching vibration)

MS: [M]⁺ = 259, [M-H]⁻ = 171

### (Synthesis Example 6) Synthesis of tetrabutylphosphonium 5-chlorosalicylate:

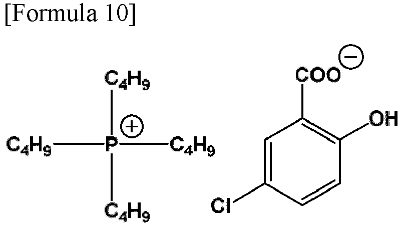

A 40% aqueous solution of tetrabutylphosphonium hydroxide (6.91 g (10 mmol)) and 5-chlorosalicylic acid (1.73 g (10 mmol)) were individually weighed and placed in a glass vial. The reaction solution was stirred at room temperature for two hours. After confirming that 5-chlorosalicylic acid was completely dissolved, the reaction was terminated. As a result, a mixture of tetrabutylphosphonium 5-chlorosalicylate and water (hereinafter referred to as Synthesis Example 6-sample) was quantitatively obtained. Synthesis Example 6-sample was used in Example 1 described later.

A part of Synthesis Example 6-sample obtained was taken and dichloromethane was added thereto to extract an organic layer. Thereafter, the organic layer was dried over anhydrous magnesium sulfate and then concentrated to obtain tetrabutylphosphonium 5-chlorosalicylate (hereinafter referred to as Synthesis Example 6-compound) as a light yellow liquid. Synthesis Example 6-compound obtained was subjected to ¹H-NMR, FT-IR and MS measurements. The measurement results are as follows. It was confirmed that a desired ionic liquid was obtained.

¹H-NMR (CDCl₃) δ: 7.87 (1H, d, J = 3.6 Hz), 7.15 (1H, dd, J = 9.0, 3.6 Hz), 6.76 (1H, d, J = 9.0 Hz), 2.27-2.16 (8H, m) 1.53-1.41 (16H, m), 0.94 (12H, t, J = 7.6 Hz).

FT-IR: 3394 cm⁻¹ (OH stretching vibration), 1577 cm⁻¹ (C=O stretching vibration)

MS: [M]⁺ = 259, [M-H]⁻ = 171

### (Synthesis Example 7) Synthesis of tetrabutylphosphonium 5-iodosalicylate:

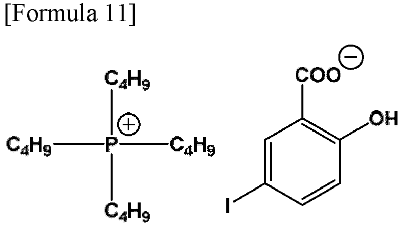

A 40% aqueous solution of tetrabutylphosphonium hydroxide (6.91 g (10 mmol)) and 5-iodosalicylic acid (2.64 g (10 mmol)) were individually weighed and placed in a glass vial. The reaction solution was stirred at room temperature for two hours. After confirming that 5-iodosalicylic acid was completely dissolved, the reaction was terminated. As a result, a mixture of tetrabutylphosphonium 5-iodosalicylate and water (hereinafter referred to as Synthesis Example 7-sample) was quantitatively obtained. Synthesis Example 7-sample was used in Example 1 described later.

A part of Synthesis Example 7-sample obtained was taken and dichloromethane was added thereto to extract an organic layer. Thereafter, the organic layer was dried over anhydrous magnesium sulfate and then concentrated to obtain tetrabutylphosphonium 5-iodosalicylate (hereinafter referred to as Synthesis Example 7-compound) as a light yellow liquid. Synthesis Example 7-compound obtained was subjected to ¹H-NMR, FT-IR and MS measurements. The measurement results are as follows. It was confirmed that a desired ionic liquid was obtained.

¹H-NMR (CDCl₃) δ: 8.19 (1H, d, J = 3.5 Hz), 7.45 (1H, dd, J = 8.5, 3.5 Hz), 6.62 (1H, d, J = 8.5 Hz), 2.40 (1H, br), 2.24-2.14 (8H, m), 1.55-1.40 (16H, m), 0.95 (12H, t, J = 7.9 Hz).

FT-IR: 3394 cm⁻¹ (OH stretching vibration), 1574 cm⁻¹ (C=O stretching vibration)

MS: [M]⁺ = 259, [M-H]⁻ = 263

### (Synthesis Example 8) Synthesis of tetrabutylphosphonium 3-carboxy-4-hydroxybenzenesulfonate:

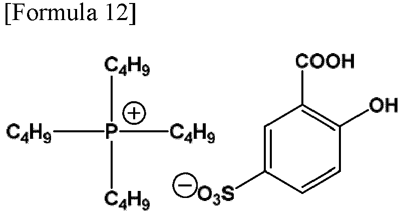

A 40% aqueous solution of tetrabutylphosphonium hydroxide (6.91 g (10 mmol)) and 5-sulfosalicylic acid dihydrate (2.54 g (10 mmol)) were individually weighed and placed in a glass vial. The reaction solution was stirred at room temperature for two hours. After confirming that 5-sulfosalicylic acid dihydrate was completely dissolved, the reaction was terminated. As a result, a mixture of tetrabutylphosphonium 3-carboxy-4-hydroxybenzenesulfonate and water (hereinafter referred to as Synthesis Example 8-sample) was quantitatively obtained. Synthesis Example 8-sample was used in Example 1 described later.

A part of Synthesis Example 8-sample obtained was taken and dichloromethane was added thereto to extract an organic layer. Thereafter, the organic layer was dried over anhydrous magnesium sulfate and then concentrated to obtain tetrabutylphosphonium 3-carboxy-4-hydroxybenzenesulfonate (hereinafter referred to as Synthesis Example 8-compound) as a light yellow liquid. Synthesis Example 8-compound obtained was subjected to ¹H-NMR and FT-IR measurements. The measurement results are as follows. It was confirmed that a desired ionic liquid was obtained.

¹H-NMR (CDCl₃) δ: 8.49 (1H, d, J = 2.8 Hz), 7.96 (1H, dd, J = 8.1, 2.8 Hz), 6.89 (1H, d, J = 8.1 Hz), 2.23-2.16 (8H, m), 1.51-1.38 (16H, m), 0.89 (12H, t, J = 7.6 Hz).

FT-IR: 3340 cm⁻¹ (OH stretching vibration), 1600 cm⁻¹ (C=O stretching vibration)

### (Synthesis Example 9) Synthesis of tetrabutylphosphonium salicylate:

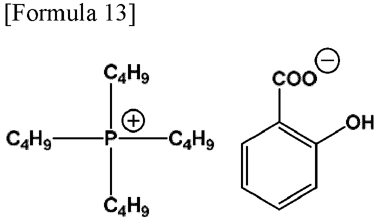

A 40% aqueous solution of tetrabutylphosphonium hydroxide (6.91 g (10 mmol)) and salicylic acid (1.38 g (10 mmol)) were individually weighed and placed in a glass vial. The reaction solution was stirred at room temperature for two hours in accordance with a method described in Chem. Commun. 10248-10250 (2013). After confirming that salicylic acid was completely dissolved, the reaction was terminated. As a result, a mixture of tetrabutylphosphonium salicylate and water (hereinafter referred to as Synthesis Example 9-sample) was quantitatively obtained. Synthesis Example 9-sample was used in Examples 1 to 6 described later.

### (Synthesis Example 10) Synthesis of tetrabutylammonium 4-(trifluoromethyl)salicylate:

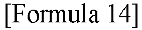

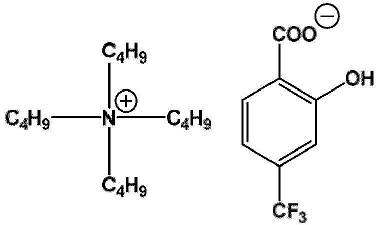

A 40% aqueous solution of tetrabutylammonium hydroxide (6.49 g (10 mmol)) and 4-(trifluoromethyl)salicylic acid (2.06 g (10 mmol)) were individually weighed and placed in a glass vial. The reaction solution was stirred at room temperature for two hours. After confirming that 4-(trifluoromethyl)salicylic acid was completely dissolved, the reaction was terminated. As a result, a mixture of tetrabutylammonium 4-(trifluoromethyl)salicylate and water (hereinafter referred to as Synthesis Example 10-sample) was quantitatively obtained. Synthesis Example 10-sample was used in Example 1 and Example 2 described later.

A part of Synthesis Example 10-sample obtained was taken and dichloromethane was added thereto to extract an organic layer. Thereafter, the organic layer was dried over anhydrous magnesium sulfate and then concentrated to obtain tetrabutylammonium 4-(trifluoromethyl)salicylate (hereinafter referred to as Synthesis Example 10-compound) as a light yellow liquid. Synthesis Example 10-compound obtained was subjected to ¹H-NMR, FT-IR and MS measurements. The measurement results are as follows. It was confirmed that a desired ionic liquid was obtained.

¹H-NMR (CDCl₃) δ: 8.02 (1H, dd, J = 8.0, 3.0 Hz), 7.05 (1H, dd, J = 3.0 Hz), 6.94 (1H, d, J = 8.0 Hz), 3.20-3.21 (8H, m) 2.53 (1H, br), 1.60-1.50 (8H, m), 1.42-1.29 (8H, m), 0.95 (12H, td, J = 7.5, 3.5 Hz).

FT-IR: 3417 cm⁻¹ (OH stretching vibration), 1593 cm⁻¹ (C=O stretching vibration)

MS: [M]⁺ = 242, [M-H]⁻ = 205

### (Synthesis Example 11) Synthesis of tetrabutylammonium salicylate:

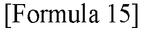

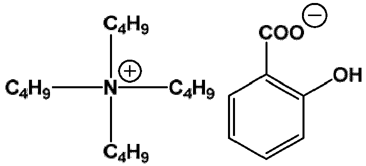

A 40% aqueous solution of tetrabutylammonium hydroxide (6.49 g (10 mmol)) and salicylic acid (1.38 g (10 mmol)) were individually weighed and placed in a glass vial. The reaction solution was stirred at room temperature for two hours. After confirming that salicylic acid was completely dissolved, the reaction was terminated. As a result, a mixture of tetrabutylammonium salicylate and water (hereinafter referred to as Synthesis Example 11-sample) was quantitatively obtained. Synthesis Example 11-sample was used in Example 1 and Example 2 described later.

### (Synthesis Example 12) Synthesis of tetrabutylphosphonium 4-chlorobenzenesulfonate:

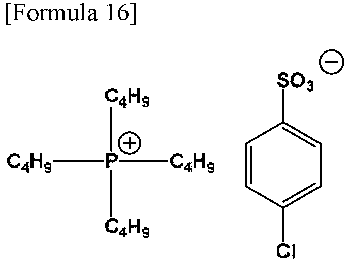

A 40% aqueous solution of tetrabutylphosphonium hydroxide (6.91 g (10 mmol)) and 4-chlorobenzenesulfonic acid hydrate (1.93 g (10 mmol)) were individually weighed and placed in a glass vial. The reaction solution was stirred at room temperature for two hours. After confirming that 4-chlorobenzenesulfonic acid hydrate was completely dissolved, the reaction was terminated. As a result, a mixture of tetrabutylphosphonium 4-chlorobenzenesulfonate and water (hereinafter referred to as Synthesis Example 12-sample) was quantitatively obtained. Synthesis Example 12-sample was used in Example 1 described later.

A part of Synthesis Example 12-sample obtained was taken and dichloromethane was added thereto to extract an organic layer. Thereafter, the organic layer was dried over anhydrous magnesium sulfate and then concentrated to obtain tetrabutylphosphonium 4-chlorobenzenesulfonate (hereinafter referred to as Synthesis Example 12-compound) as a light yellow liquid. Synthesis Example 12-compound was subjected to ¹H-NMR and MS measurements. The measurement results are as follows. It was confirmed that a desired ionic liquid was obtained.

¹H-NMR (CDCl₃) δ: 7.84 (2H, d, J = 8.0 Hz), 7.30 (1H, d, J = 8.0 Hz), 2.33-2.25 (8H, m), 1.56-1.42 (16H, m), 0.96 (12H, t, J = 8.2 Hz).

MS: [M]⁺ = 259, [M-H]⁻ = 191

### (Synthesis Example 13) Synthesis of tetrabutylphosphonium 4-hydroxybenzenesulfonate:

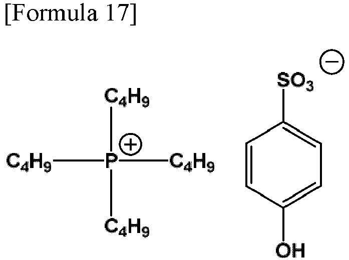

A 40% aqueous solution of tetrabutylphosphonium hydroxide (6.91 g (10 mmol)) and 4-hydroxybenzenesulfonic acid hydrate (1.74 g (10 mmol)) were individually weighed and placed in a glass vial. The reaction solution was stirred at room temperature for two hours. After confirming that 4-hydroxybenzenesulfonic acid hydrate was completely dissolved, the reaction was terminated. As a result, a mixture of tetrabutylphosphonium 4-hydroxybenzenesulfonate and water (hereinafter referred to as Synthesis Example 13-sample) was quantitatively obtained. Synthesis Example 13-sample was used in Example 1 described later.

A part of Synthesis Example 13-sample obtained was taken and dichloromethane was added thereto to extract an organic layer. Thereafter, the organic layer was dried over anhydrous magnesium sulfate and then concentrated to obtain tetrabutylphosphonium 4-hydroxybenzenesulfonate (hereinafter referred to as Synthesis Example 13-compound) as a light yellow liquid. Synthesis Example 13-compound obtained was subjected to ¹H-NMR measurement. The measurement results are as follows. It was confirmed that a desired ionic liquid was obtained.

¹H-NMR (CDCl₃) δ: 7.59 (2H, d, J = 8.8 Hz), 6.89 (1H, d, J = 8.8 Hz), 2.08-1.98 (8H, m), 1.47-1.22 (16H, m), 0.89 (12H, t, J = 7.7 Hz).

### (Synthesis Example 14) Synthesis of tetrabutylphosphonium 4-aminotoluene-3-sulfonate:

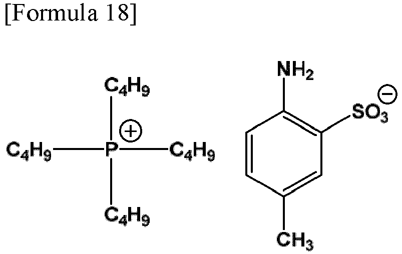

A 40% aqueous solution of tetrabutylphosphonium hydroxide (6.91 g (10 mmol)) and 4-aminotoluene-3-sulfonic acid (1.87 g (10 mmol)) were individually weighed and placed in a glass vial. The reaction solution was stirred at room temperature for two hours. After confirming that 4-aminotoluene-3-sulfonic acid was completely dissolved, the reaction was terminated. As a result, a mixture of tetrabutylphosphonium 4-aminotoluene-3-sulfonate and water (hereinafter referred to as Synthesis Example 14-sample) was quantitatively obtained. Synthesis Example 14-sample was used in Example 1 described later.

A part of Synthesis Example 14-sample obtained was taken and dichloromethane was added thereto to extract an organic layer. Thereafter, the organic layer was dried over anhydrous magnesium sulfate and then concentrated to obtain tetrabutylphosphonium 4-aminotoluene-3-sulfonate (hereinafter referred to as Synthesis Example 14-compound) as a yellow viscous liquid. Synthesis Example 14-compound obtained was subjected to ¹H-NMR measurement. The measurement results are as follows. It was confirmed that a desired ionic liquid was obtained.

¹H-NMR (CDCl₃) δ: 7.59 (1H, d, J = 2.5 Hz), 6.89 (1H, dd, J = 8.2, 2.5 Hz), 6.54 (1H, d, J = 8.2 Hz), 2.28-2.21 (8H, m), 2.19 (3H, s), 1.52-1.40 (16H, m), 0.94 (12H, t, J = 7.5 Hz).

### (Synthesis Example 15) Synthesis of tetrabutylphosphonium m-xylene-4-sulfonate:

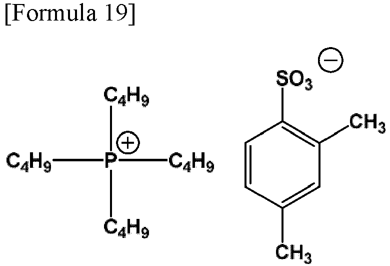

A 40% aqueous solution of tetrabutylphosphonium hydroxide (6.91 g (10 mmol)) and m-xylene-4-sulfonic acid hydrate (1.86 g (10 mmol)) were individually weighed and placed in a glass vial. The reaction solution was stirred at room temperature for two hours. After confirming that m-xylene-4-sulfonic acid hydrate was completely dissolved, the reaction was terminated. As a result, a mixture of tetrabutylphosphonium m-xylene-4-sulfonate and water (hereinafter referred to as Synthesis Example 15-sample) was quantitatively obtained. Synthesis Example 15-sample was used in Example 1 and Example 2 described later.

### (Synthesis Example 16) Synthesis of tetrabutylphosphonium p-toluenesulfonate:

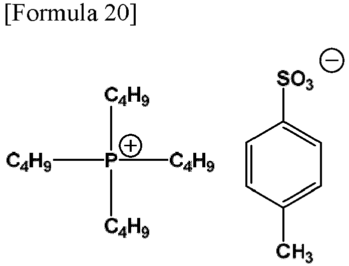

A 40% aqueous solution of tetrabutylphosphonium hydroxide (6.91 g (10 mmol)) and p-toluenesulfonic acid monohydrate (1.90 g (10 mmol)) were individually weighed and placed in a glass vial. The reaction solution was stirred at room temperature for two hours. After confirming that p-toluenesulfonic acid monohydrate was completely dissolved, the reaction was terminated. As a result, a mixture of tetrabutylphosphonium p-toluenesulfonate and water (hereinafter referred to as Synthesis Example 16-sample) was quantitatively obtained. Synthesis Example 16-sample was used in Example 1 described later.

### (Synthesis Example 17) Synthesis of tetrabutylphosphonium salicylate/p-toluenesulfonate:

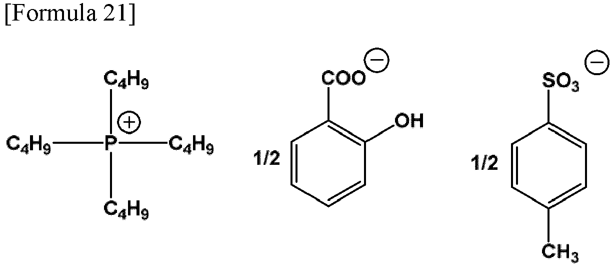

A 40% aqueous solution of tetrabutylphosphonium hydroxide (6.91 g (10 mmol)), salicylic acid (0.69 g (5 mmol)) and p-toluenesulfonic acid monohydrate (0.95 g (5 mmol)) were individually weighed and placed in a glass vial. The reaction solution was stirred at room temperature for two hours. After confirming that salicylic acid and p-toluenesulfonic acid monohydrate were completely dissolved, the reaction was terminated. As a result, a mixture of tetrabutylphosphonium salicylate/p-toluenesulfonate and water (hereinafter referred to as Synthesis Example 17-sample) was quantitatively obtained. Synthesis Example 17-sample was used in Example 1 described later.

### (Reference Example 1) Synthesis of tetrabutylphosphonium 4-aminosalicylate:

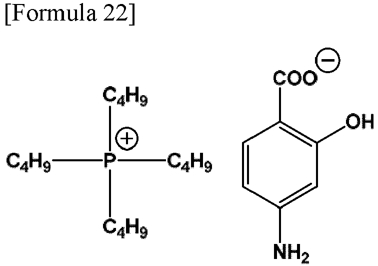

A 40% aqueous solution of tetrabutylphosphonium hydroxide (6.91 g (10 mmol)) and 4-aminosalicylic acid (1.53 g (10 mmol)) were individually weighed and placed in a glass vial. The reaction solution was stirred at room temperature for two hours. After confirming that 4-aminobenzenesulfonic acid was completely dissolved, the reaction was terminated. As a result, a mixture of tetrabutylphosphonium 4-aminobenzenesulfonate and water (hereinafter referred to as Reference Example 1-sample) was quantitatively obtained. Reference Example 1-sample was used in Example 1 described later.

### (Synthesis Example 18) Synthesis of tetrabutylphosphonium salicylate/4-(trifluoromethyl)salicylate (1:1):

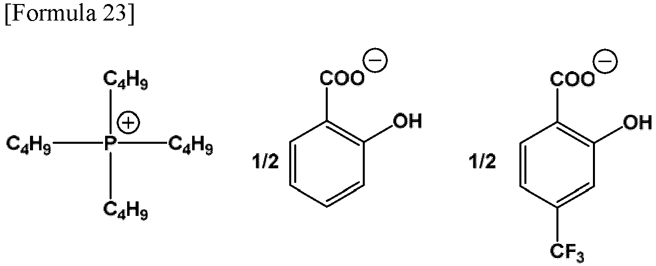

A 40% aqueous solution of tetrabutylphosphonium hydroxide (6.91 g (10 mmol)), salicylic acid (0.69 g (5 mmol)) and 4-(trifluoromethyl)salicylic acid (1.03 g (5 mmol)) were individually weighed and placed in a glass vial. The reaction solution was stirred at room temperature for two hours. After confirming that salicylic acid and 4-(trifluoromethyl)salicylic acid were completely dissolved, the reaction was terminated. As a result, a mixture of tetrabutylphosphonium salicylate/4-(trifluoromethyl)salicylate and water (hereinafter referred to as Synthesis Example 18-sample) was quantitatively obtained. Synthesis Example 18-sample was used in Example 1 described later.

### (Synthesis Example 19) Synthesis of tetrabutylphosphonium salicylate/4-(trifluoromethyl)salicylate (2:1):

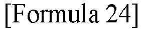

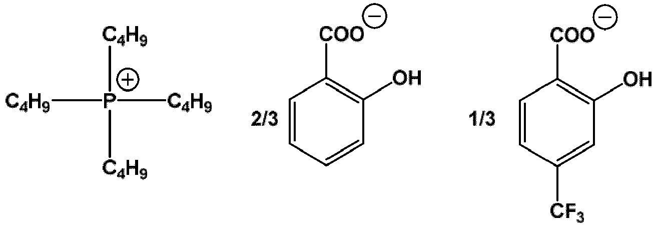

A 40% aqueous solution of tetrabutylphosphonium hydroxide (6.91 g (10 mmol)), salicylic acid (0.92 g (6.7 mmol)) and 4-(trifluoromethyl)salicylic acid (0.69 g (3.3 mmol)) were individually weighed and placed in a glass vial. The reaction solution was stirred at room temperature for two hours. After confirming that salicylic acid and 4-(trifluoromethyl)salicylic acid were completely dissolved, the reaction was terminated. As a result, a mixture of tetrabutylphosphonium salicylate/4-(trifluoromethyl)salicylate (2:1) and water (hereinafter referred to as Synthesis Example 19-sample) was quantitatively obtained. Synthesis Example 19-sample was used in Example 1 and Example 2 described later.

### (Synthesis Example 20) Synthesis of tetrabutylphosphonium salicylate/4-(trifluoromethyl)salicylate (3:1):

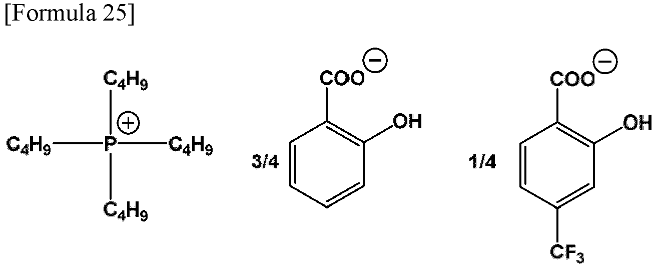

A 40% aqueous solution of tetrabutylphosphonium hydroxide (6.91 g (10 mmol)), salicylic acid (1.04 g (7.5 mmol)) and 4-(trifluoromethyl)salicylic acid (0.690.52 g (2.5 mmol)) were individually weighed and placed in a glass vial. The reaction solution was stirred at room temperature for two hours. After confirming that salicylic acid and 4-(trifluoromethyl)salicylic acid were completely dissolved, the reaction was terminated. As a result, a mixture of tetrabutylphosphonium salicylate/4-(trifluoromethyl)salicylate and water (hereinafter referred to as Synthesis Example 20-sample) was quantitatively obtained. Synthesis Example 20-sample was used in Example 1 described later.

### (Reference Example 2) Synthesis of tetrabutylphosphonium benzenesulfonate:

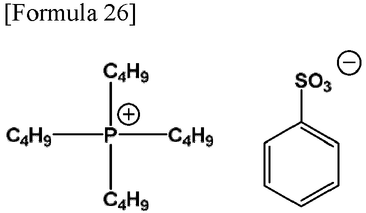

A 40% aqueous solution of tetrabutylphosphonium hydroxide (6.91 g (10 mmol)) and benzenesulfonic acid monohydrate (1.76 g (10 mmol)) were individually weighed and placed in a glass vial. The reaction solution was stirred at room temperature for two hours. After confirming that benzenesulfonic acid monohydrate was completely dissolved, the reaction was terminated. As a result, a mixture of tetrabutylphosphonium benzenesulfonate and water (hereinafter referred to as Reference Example 2-sample) was quantitatively obtained. Reference Example 2-sample was used in Example 1 and Example 2 described later.

### (Reference Example 3) Synthesis of cholinium decanoate:

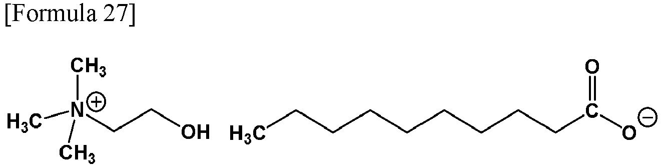

A 50% aqueous solution of choline (2.42 g (10 mmol)), decanoic acid (1.72 g (10 mmol)) and Milli-Q (registered trademark, Merck KGaA) water (4.5 mL) were individually weighed and placed in a glass vial. The reaction solution was stirred at room temperature for two hours. After confirming that decanoic acid was completely dissolved, the reaction was terminated. As a result, a mixture of cholinium decanoate and water (hereinafter referred to as Reference Example 3-sample) was quantitatively obtained. Reference Example 3-sample was used in Example 1 and Example 2 described later. Note that, cholinium decanoate corresponds to choline-capric acid described in Patent Literature 3.

### (Reference Example 4) Synthesis of diisopropanolammonium decanoate:

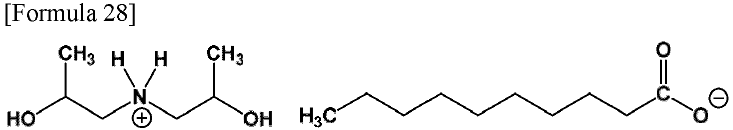

A 20% aqueous solution of diisopropanolamine (6.65 g (10 mmol)) and decanoic acid (.72 g (10 mmol)) were individually weighed and placed in a glass vial. The reaction solution was stirred at room temperature for two hours. After confirming that decanoic acid was completely dissolved, the reaction was terminated. As a result, a mixture of diisopropanolammonium decanoate and water (hereinafter referred to as Reference Example 4-sample) was quantitatively obtained. Reference Example 4-sample was used in Example 1 and Example 2 described later. Note that, diisopropanolammonium decanoate corresponds to capric acid diisopropanolamine salt described in Patent Literature 1.

### (Reference Example 5) Synthesis of cholinium salicylate:

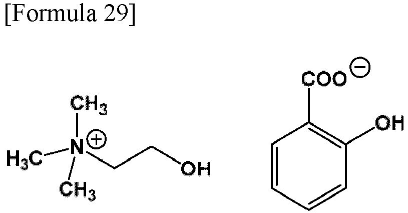

A 50% aqueous solution of choline (2.42 g (10 mmol)), salicylic acid (1.38 g (10 mmol)) and Milli-Q (registered trademark, Merck KGaA) water (4.5 mL) were individually weighed and placed in a glass vial. The reaction solution was stirred at room temperature for two hours. After confirming that salicylic acid was completely dissolved, the reaction was terminated. As a result, a mixture of cholinium salicylate and water (hereinafter referred to as Reference Example 5-sample) was quantitatively obtained. Reference Example 5-sample was used in Example 1 described later.

### (Example 1) Confirmation of temperature responsive property of ionic liquid

Synthesis Example 1 to 20-samples, Reference Example 1 to 5-samples, a mixture of Reference Example 1-sample and Synthesis Example 9-sample (volume ratio 1:1), a mixture of Reference Example 1-sample and Synthesis Example 3-sample (volume ratio 1:1), a mixture of Reference Example 1-sample and Synthesis Example 4-sample (volume ratio 1:1), mixtures of Synthesis Example 9-sample and Synthesis Example 16-sample (volume ratio 1:1) and (volume ratio 2:1) were examined as to whether they have a temperature responsive property or not. More specifically, the temperature responsive property was examined by measuring a absorbance at 450 nm at different temperatures in the following measurement conditions to obtain a change in absorbance. Note that, in the cases of ionic liquids (Synthesis Example 3-sample, Synthesis Example 4-sample, Synthesis Example 9-sample, Synthesis Example 11-sample, Synthesis Example 14-sample, Synthesis Example 16-sample, Synthesis Example 17-sample, Reference Example 1 to 5-samples) that are compatible with water at room temperature and mixtures thereof, aqueous solutions of ionic liquids were directly measured. In contrast, in the cases of ionic liquids (Synthesis Example 1-sample, Synthesis Example 2-sample, Synthesis Example 5 to 8-samples, Synthesis Example 10-sample, Synthesis Example 12-sample, Synthesis Example 13-sample, Synthesis Example 15-sample), which were separated from water at room temperature, mixtures of water and the ionic liquids described in the respective Synthesis Examples were subjected to centrifugation or allowed to stand still. The resultant ionic liquid layers separated were taken out and subjected to measurement. Synthesis Example 18 to 20-samples were allowed to stand still in a refrigerator of 4°C for one hour or more, stirred at 4°C, and again allowed to stand still in a refrigerator of 4°C. The resultant homogeneous samples were subjected to measurement. The results are shown in Table 1-1, Table 1-2 and Table 1-3. It was confirmed that the ionic liquid samples except Reference Example 1 to 5-samples are temperature-responsive ionic liquid having a temperature responsive property with a lower critical solution temperature.

### (Measurement conditions)

- Measuring device: spectrophotometer UV1650PC manufactured by Shimadzu Corporation
- Detection wavelength: 450 nm
- Initial waiting time: 10 minutes
- Heating condition: 20°C → 95°C (0.5°C/minute)
- Analysis method: calculation by differential calculus using Tm (nucleic acid melting temperature) analysis program

Note that, measurement of Reference Example 3 to 5-samples was carried out in the following conditions.
- Measuring device: spectrophotometer V-750 manufactured by JASCO Corporation
- Detection wavelength: 450 nm
- Initial waiting time: 5 minutes
- Heating conditions: 20°C → 95°C (0.5°C/minute)
- Analysis method: calculation by differential calculus using Tm (nucleic acid melting temperature) analysis program

Measurement of Synthesis Example 18 to 20-samples were carried out in the following conditions.
- Measuring device: spectrophotometer V-750 manufactured by JASCO Corporation
- Detection wavelength: 450 nm
- Initial waiting time: 10 minutes
- Heating conditions: 5°C → 40°C (0.5°C/minute)
- Analysis method: calculation by differential calculus using Tm (nucleic acid melting temperature) analysis program

**[Table 1-1]**

| Test sample | Ionic liquid | LCST (°C) |
|---|---|---|
| Synthesis Example 1-sample | Tetrabutylphosphonium 4-(trifluoromethyl)salicylate | 23.6 |
| Synthesis Example 2-sample | Tetrabutylphosphonium 5-bromosalicylate | 27.7 |
| Synthesis Example 3-sample | Tetrabutylphosphonium 3-methylsalicylate | 23.5 |
| Synthesis Example 4-sample | Tetrabutylphosphonium 4-methylsalicylate | 24.5 |
| Synthesis Example 5-sample | Tetrabutylphosphonium 4-chlorosalicylate | 29.7 |
| Synthesis Example 6-sample | Tetrabutylphosphonium 5-chlorosalicylate | 32.4 |
| Synthesis Example 7-sample | Tetrabutylphosphonium 5-iodosalicylate | 32.7 |
| Synthesis Example 8-sample | Tetrabutylphosphonium 3-carboxy-4-hydroxybenzenesulfonate | 29.6 |
| Synthesis Example 9-sample | Tetrabutylphosphonium salicylate | 25.7 |
| Synthesis Example 10-sample | Tetrabutylammonium 4-(trifluoromethyl)salicylate | 23.7 |
| Synthesis Example 11-sample | Tetrabutylammonium salicylate | 37.2 |
| Synthesis Example 12-sample | Tetrabutylphosphonium 4-chlorobenzenesulfonate | 25.6 |
| Synthesis Example 13-sample | Tetrabutylphosphonium 4-hydroxybenzenesulfonate | 24.7 |
| Synthesis Example 14-sample | Tetrabutylphosphonium 4-aminotoluene-3-sulfonate | 41.4 |
| Synthesis Example 15-sample | Tetrabutylphosphonium m-xylene-4-sulfonate | 23.7 |
| Synthesis Example 16-sample | Tetrabutylphosphonium p-toluene sulfonate | 54.6 |
| Synthesis Example 17-sample | Tetrabutylphosphonium salicylate·p-toluene sulfonate | 32.6 |

**[Table 1-2]**

| Test sample | Ionic liquid | LCST (°C) |
|---|---|---|
| Mixture of Reference Example 1-sample + Synthesis Example 9-sample (1:1) | Mixture of tetrabutylphosphonium 4-aminosalicylate + tetrabutylphosphonium salicylate (1:1) | 54.6 |
| Mixture of Reference Example 1-sample + Synthesis Example 3-sample (1:1) | Mixture of tetrabutylphosphonium 4-aminosalicylate + tetrabutylphosphonium 3-methylsalicylate (1:1) | 49.7 |
| Mixture of Reference Example 1-sample + Synthesis Example 4-sample (1:1) | Mixture of tetrabutylphosphonium 4-aminosalicylate + tetrabutylphosphonium 4-methylsalicylate (1:1) | 51.6 |
| Mixture of Synthesis Example 9-sample + Synthesis Example 16-sample (1:1) | Mixture of Tetrabutylphosphonium salicylate + tetrabutylphosphonium p-toluene sulfonate (1:1) | 32.6 |
| Mixture of Synthesis Example 9-sample + Synthesis Example 16-sample (2:1) | Mixture of Tetrabutylphosphonium salicylate + tetrabutylphosphonium p-toluenesulfonate (2:1) | 29.6 |
| Reference Example 1-sample | Tetrabutylphosphonium 4-aminosalicylate | None |

**[Table 1-3]**

| Test sample | Ionic liquid | LCST (°C) |
|---|---|---|
| Synthesis Example 18-sample | Tetrabutylphosphonium salicylate/4-(trifluoromethyl)salicylate (1:1) | 17.8 |
| Synthesis Example 19-sample | Tetrabutylphosphonium salicylate/4-(trifluoromethyl)salicylate (2:1) | 18.9 |
| Synthesis Example 20-sample | Tetrabutylphosphonium salicylate/4-(trifluoromethyl)salicylate (3:1) | 18.9 |
| Reference Example 2-sample | Tetrabutylphosphonium benzenesulfonate | None |
| Reference Example 3-sample | Cholinium decanoate | None |
| Reference Example 4-sample | Diisopropanolammonium decanoate | None |
| Reference Example 5-sample | Cholinium salicylate | None |

In Table 1-1, Table 1-2 and Table 1-3, LCST represents the lower critical solution temperature.

### (Example 2) Permeation test of pregabalin using human cultured epidermal model

A human cultured epidermal model (LabCyte EPI-MODEL12, Japan Tissue Engineering Co., Ltd.) was used. The phase on the stratum corneum side of the model was defined as a donor phase, whereas the phase on the stratum basal side of the model was defined as a receptor phase. Whether a temperature-responsive ionic liquid enhances the permeation of pregabalin from the donor phase to the receptor phase was examined. The test was carried out in an incubator of 37°C. Pregabalin was dissolved in water, or a 100 mmol/L aqueous solution or water dispersion of a temperature-responsive ionic liquid to a concentration of 10 mg/mL. Each solution (300 µL) was added to the donor phase. Twenty-four hours later, a receptor fluid was sampled and analyzed by LC-MS/MS to obtain the amount of pregabalin permeated. Note that, the Hanks' balanced salt solution was used as the receptor fluid. As test samples, Synthesis Example 1-sample, Synthesis Example 9-sample, Synthesis Example 10-sample, Synthesis Example 11-sample, Synthesis Example 15-sample, Synthesis Example 19-sample and Reference Example 2 to 4-samples were used at the above concentration.

The results are shown in Table 2. It was confirmed that in the cases where pregabalin was dissolved in a temperature-responsive ionic liquid, the permeation amount of pregabalin (CLogP=-0.516), a water-soluble drug which is low in skin permeability, was remarkably high compared to the control case where pregabalin was dissolved in water. It was demonstrated that skin permeability of pregabalin is enhanced. Particularly, the effect of temperature-responsive ionic liquids having an anion derived from salicylic acid or a salicylic acid derivative was high. In contrast, the cases where Reference Example 2 to 4-samples were used, the effect of enhancing skin permeation of pregabalin was low compared to the cases where temperature-responsive ionic liquids were used.

**[Table 2]**

| Test sample | Temperature-responsive ionic liquid or ionic liquid | 24-hour Cumulative permeation amount (µg/cm²) |
|---|---|---|
| Synthesis Example 1-sample | Tetrabutylphosphonium 4-(trifluoromethyl)salicylate | 3030 |
| Synthesis Example 9-sample | Tetrabutylphosphonium salicylate | 3410 |
| Synthesis Example 10-sample | Tetrabutylammonium 4-(trifluoromethyl)salicylate | 3480 |
| Synthesis Example 11-sample | Tetrabutylammonium salicylate | 1120 |
| Synthesis Example 15-sample | Tetrabutylphosphonium m-xylene-4-sulfonate | 1100 |
| Synthesis Example 19-sample | Tetrabutylphosphonium salicylate/4-(trifluoromethyl)salicylate (2:1) | 4150 |
| Reference Example 2-sample | Tetrabutylphosphonium benzenesulfonate | 468 |
| Reference Example 3-sample | Cholinium decanoate | 354 |
| Reference Example 4-sample | Diisopropanolammonium decanoate | 493 |
| Control | none | 252 |

### (Example 3) Permeation test of etodolac using human cultured epidermal model

A human cultured epidermal model (LabCyte EPI-MODEL12, Japan Tissue Engineering Co., Ltd.) was used. The phase on the stratum corneum side of the model was defined as a donor phase, whereas the phase on the stratum basal side of the model was defined as a receptor phase. Whether a temperature-responsive ionic liquid enhances the permeation of etodolac from the donor phase to the receptor phase was examined. The test was carried out in an incubator of 37°C. Etodolac was suspended in a 0.5% aqueous solution of methyl cellulose or dissolved in a 100 mmol/L aqueous solution of a temperature-responsive ionic liquid. Each solution (300 µL) was added to the donor phase. Twenty-four hours later, a receptor fluid was sampled and analyzed by LC-MS/MS to obtain the amount of etodolac permeated. Note that, the Hanks' balanced salt solution was used as the receptor fluid. As test samples, Synthesis Example 9-sample was used at the above concentration.

The results are shown in Table 3. In the case where a lipophilic drug, etodolac (CLogP=3.43), the permeation amount of etodolac was also increased by use of a temperature-responsive ionic liquid of Synthesis Example 9. Note that, in the case (control) where a 0.5% aqueous solution of methyl cellulose was used, a part of etodolac was not dissolved and became a suspension. In contrast, in the case where the temperature-responsive ionic liquid of Synthesis Example 9 was used, an etodolac solution is resulted. It became possible to completely dissolve etodolac. From this, it was demonstrated that the solubility of a drug such as etodolac can be improved by use of a temperature-responsive ionic liquid. It was thus confirmed that both the permeability of a drug to the skin and the solubility of a drug can be improved.

**[Table 3]**

| Test sample | Temperature-responsive ionic liquid | 24-hour Cumulative permeation amount (µg/cm²) | Etodolac solubility |
|---|---|---|---|
| Synthesis Example 9-sample | Tetrabutylphosphonium salicylate | 42.5 | ○ (homogeneous solution) |
| Control | None | 34.9 | × (suspension) |

### (Example 4) Permeation test of lidocaine hydrochloride monohydrate using human cultured epidermal model

A human cultured epidermal model (LabCyte EPI-MODEL12, Japan Tissue Engineering Co., Ltd.) was used. The phase on the stratum corneum side of the model was defined as a donor phase, whereas the phase on the stratum basal side of the model was defined as a receptor phase. Whether a temperature-responsive ionic liquid enhances the permeation of lidocaine hydrochloride monohydrate from the donor phase to the receptor phase was examined. The test was carried out in an incubator of 37°C. Lidocaine hydrochloride monohydrate was dissolved in water, or a 100 mmol/L aqueous solution or water dispersion of a temperature-responsive ionic liquid to a concentration of 10 mg/mL. Each solution (300 µL) was added to the donor phase. Twenty-four hours later, a receptor fluid was sampled and analyzed by LC-MS/MS to obtain the amount of lidocaine hydrochloride monohydrate permeated. Note that, the Hanks' balanced salt solution was used as the receptor fluid. As test samples, Synthesis Example 1-sample and Synthesis Example 9-sample were used at the above concentration.

The results are shown in Table 4. It was confirmed that in the cases where lidocaine (CLogP=1.95) hydrochloride monohydrate (a basic drug) was dissolved in the temperature-responsive ionic liquid, the amount of lidocaine hydrochloride monohydrate permeated through the skin is also greatly improved, compared to the case (control) where lidocaine hydrochloride monohydrate was dissolved in water.

**[Table 4]**

| Test sample | Temperature-responsive ionic liquid | 24-hour Cumulative permeation amount (µg/cm²) |
|---|---|---|
| Synthesis Example 1-sample | Tetrabutylphosphonium 4-(trifluoromethyl)salicylate | 1100 |
| Synthesis Example 9-sample | Tetrabutylphosphonium salicylate | 829 |
| Control | None | 308 |

### (Example 5) Permeation test of tramadol hydrochloride using human cultured epidermal model

A human cultured epidermal model (LabCyte EPI-MODEL12, Japan Tissue Engineering Co., Ltd.) was used. The phase on the stratum corneum side of the model was defined as a donor phase, whereas the phase on the stratum basal side of the model was defined as a receptor phase. Whether a temperature-responsive ionic liquid enhances the permeation of tramadol hydrochloride from the donor phase to the receptor phase was examined. The test was carried out in an incubator of 37°C. Tramadol hydrochloride was dissolved in water, or a 100 mmol/L aqueous solution or water dispersion of a temperature-responsive ionic liquid to a concentration of 10 mg/mL. Each solution (300 µL) was added to the donor phase. Twenty-four hours later, a receptor fluid was sampled and analyzed by LC-MS/MS to obtain the amount of tramadol hydrochloride permeated. Note that, the Hanks' balanced salt solution was used as the receptor fluid. As test samples, Synthesis Example 1-sample and Synthesis Example 9-sample were used at the above concentration.

**[Table 5]**

| Test sample | Temperature-responsive ionic liquid | 24-hour Cumulative permeation amount (µg/cm²) |
|---|---|---|
| Synthesis Example 1-sample | Tetrabutylphosphonium 4-(trifluoromethyl)salicylate | 2510 |
| Synthesis Example 9-sample | Tetrabutylphosphonium salicylate | 4030 |
| Control | None | 300 |

The results are shown in Table 5. It was confirmed that in the cases where a basic drug, tramadol (CLogP=3.10) hydrochloride, was dissolved in the temperature-responsive ionic liquid, the amount of tramadol hydrochloride permeated through the skin is also greatly improved compared to the case (control) where tramadol hydrochloride was dissolved in water.

### (Example 6) Preparation of etodolac oral preparation

Etodolac (0.287 mg) was mixed with 1 mL of a 1 mol/L aqueous solution of tetrabutylphosphonium salicylate (Synthesis Example 9-sample) in a mortar to prepare a liquidstate etodolac oral preparation. As a control, etodolac (0.287 mg) and water (1 mL) were mixed in a mortar. In this case, a homogenous solution was not obtained but a suspension was only obtained. Although the solubility of etodolac to water is 0.11 mg/mL, a homogenous oral preparation containing etodolac in a higher concentration (> 0.287 mg/mL) can be obtained by use of tetrabutylphosphonium salicylate (Synthesis Example 9-sample).

From the results, it was demonstrated that the solubility of a drug can be improved by use of a temperature-responsive ionic liquid, and that regardless of the structure or physical properties of the drug the permeability of a drug can be improved by adding a temperature-responsive ionic liquid to a pharmaceutical composition.

### Industrial Applicability

The pharmaceutical composition of the present invention can improve the permeability of a drug to the skin and mucous membrane and thus can be used as a pharmaceutical drug such as an external agent.

## Claims

1. A pharmaceutical composition comprising a drug and a temperature-responsive ionic liquid.

2. The pharmaceutical composition according to claim 1, wherein an anion in the temperature-responsive ionic liquid includes at least one selected from the group consisting of an aromatic carboxylate ion and an aromatic sulfonate ion.

3. The pharmaceutical composition according to claim 1 or 2, wherein the anion in the temperature-responsive ionic liquid is an anion represented by the following formula (I) and/or general formula (II): 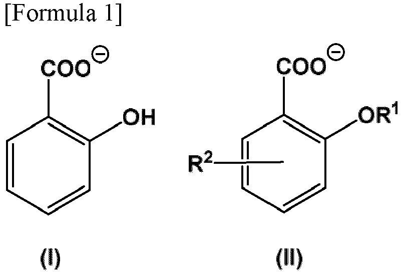 wherein in the formula (II), R¹ represents a hydrogen atom or an acyl group and R² represents an alkyl group optionally substituted with a halogen atom, a halogen atom or a sulfo group.

4. The pharmaceutical composition according to any one of claims 1 to 3, wherein the temperature-responsive ionic liquid has a lower critical solution temperature.

5. The pharmaceutical composition according to claim 4, wherein the lower critical solution temperature is 40°C or less

6. The pharmaceutical composition according to any one of claims 1 to 5, wherein a cation in the temperature-responsive ionic liquid is a tetraalkylammonium ion or a tetraalkylphosphonium ion.

7. The pharmaceutical composition according to any one of claims 1 to 6, which is an external agent.

8. A temperature-responsive ionic liquid having an anion represented by the following general formula (II): 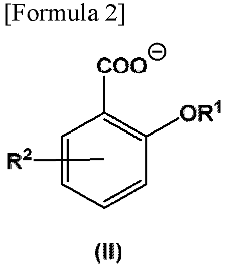 wherein in the formula (II), R¹ represents a hydrogen atom or an acyl group and R² represents an alkyl group optionally substituted with a halogen atom, a halogen atom or a sulfo group.

9. The temperature-responsive ionic liquid according to claim 8, having a lower critical solution temperature.
